# EUROPEAN PATENT APPLICATION

(11) **EP 1 393 647 A1**
(43) Date of publication of application: **03.03.2004**
(21) Application number: 03077613.2
(22) Date of filing: 21.08.2003
(51) Int. Cl.: A45C 11/00, A47G 23/02

(54) **A holder for a bottle intended for containing a fluid to be used for cleaning and maintaining contact lenses**

(30) Priority: 26.08.2002 NL 1021327
(71) Applicant: Oté Pharma Sol B.V., 5406 XP Uden (NL)
(72) Inventor: Oorthuizen, Rob Willem, 5403 TR Uden (NL)
(74) Representative: Valkonet, Rutger

(57) **Abstract**

A holder for a bottle, which bottle is intended for containing a fluid to be used for cleaning contact lenses, said holder (1,11,21) defining at least two partial surfaces (2+5;12+15;22+25) and, in addition to that, comprising two upright walls (3+4;13+14;23+24). The invention furthermore relates to a combination of the aforesaid holder (1,11,21) and a bottle, which bottle is intended for containing a fluid.

## Description

### Field of the Invention

The invention relates to a holder for a bottle intended for containing a fluid to be used for cleaning and maintaining contact lenses, which holder defines at least two partial surfaces and which comprises two upright walls.

The invention furthermore relates to a combination of a bottle and a holder, which bottle is intended for containing a fluid to be used for cleaning and maintaining contact lenses.

A holder for a bottle and a combination of a bottle and a holder is known from US patent No. 2,264,489. The holder disclosed therein is made of a flexible material, with flexible fingers functioning to ensure the upright position of the bottle. One drawback of this known combination of a bottle and a holder, however, is the fact that it is not very stable in the upright position of the bottle, due to the use of the flexible material, so that there is considerable risk of the bottle falling over.

US patent No. 3,326,358 is furthermore aimed at providing a solution to the problem of storing the lens cl eani ng fluid and the contact lenses themselves in close proximity of each other. It does not deal with the problem that is pointed out herein, viz. increasing the stability of the bottle.

A solution to the problem as indicated in US patent No. 3,326,358 is also proposed in US patents Nos. 4,925,017 and 6,073,757. The bottle disclosed in US 6,073,757 does not offer a solution to the stability problem, either. According to US patent No. 4,925,017, the bottle cannot fall over, to be true, but a new problem arises in the sense that the fluid will leak from the bottle if the bottle is not closed properly, so that the solution proposed therein is not the right solution to the aforesaid problem of preventing the bottle containing the fluid for cleaning and maintaining - in the widest sense of the word - contact lenses from falling over. The creation of a different bottle design, in which the base area of the bottle is larger than the side wall area, is not described therein. Consequently, a solution to the problem of increasing the stability of the bottle during storage thereof as referred to in the introduction is proposed herein.

In the prior art, holders for displaying medicines contained in small bottles are furthermore known; European patent application No. 0 254 487 A2 discloses such a holder, which holder is so designed that the bottles are stored at an angle so as to enable easy reading of the label. In the present case, however, it is preferable to store the bottle in an upright position.

The holder now being developed increases the stability of the bottle in the upright position to a significant extent, so that it will be easier for the consumer to handle the bottle, as a result of which the bottle is prevented from falling over. Furthermore, such a holder can be successfully used in the sale of such bottles with fluid in them.

The present invention therefore provides a holder for a bottle intended for containing a fluid to be used for cleaning contact lenses, which holder defines at least two partial surfaces and which comprises two upright walls, characterized in that the first partial surface extends parallel to a base defined by the bottom of the bottle to be placed, wherein at least two rigid upright walls extend from said first partial surface for stabilising the bottle, and wherein moreover the surface area of the second partial surface amounts to at least 50% of the surface area of the first partial surface.

Special embodiments of the present invention are defined in the appended claims.

The invention will be explained in more detail hereinafter with reference to the appended drawing, in which:
Figure 1 is a perspective view of a first embodiment of the holder according to the invention;
Figure 2 shows the holder of Figure 1 with objects present therein or thereon;
Figure 3 is a perspective view of a second embodiment of the invention;
Figure 4a is a schematic cross-sectional view of the holder of Figure 3;
Figure 4b is a schematic cross-sectional view of a simplified embodiment of the holder of Figure 3;
Figure 5 shows the holder of Figure 3 with objects present therein or thereon;
Figure 6 is a perspective view of a third embodiment of the invention;
Figure 7 is a schematic cross-sectional view of the holder of Figure 6; and
Figure 8 shows the holder of Figure 6 with objects present therein or thereon.

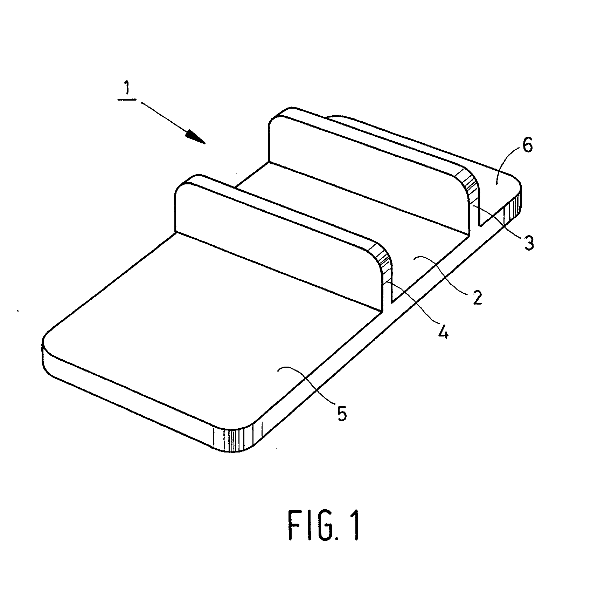

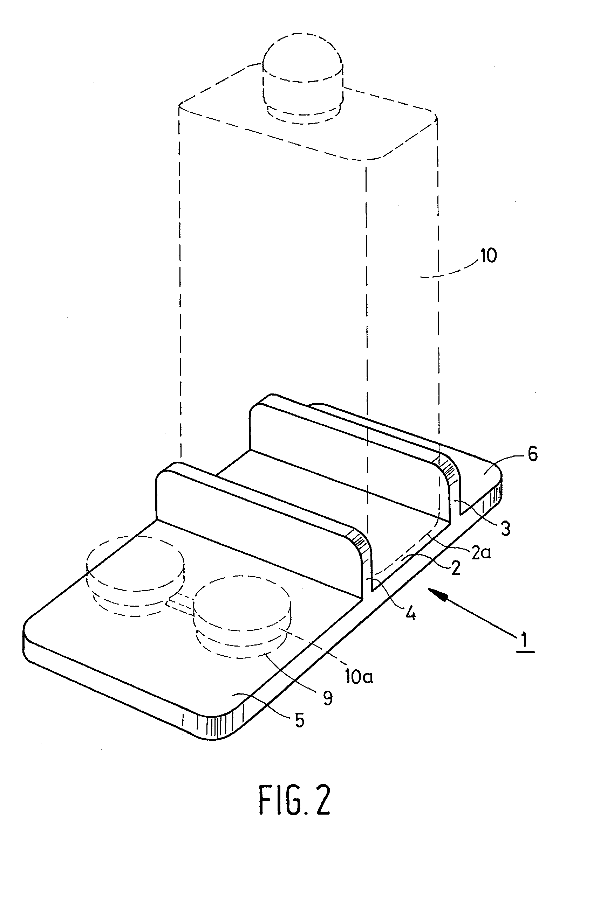

In Figures 1 and 2, numeral 1 indicates the holder according to one embodiment of the invention. The holder 1 comprises at least two partial surfaces, viz. the first partial surface 2 and the second partial surface 5, which two partial surfaces 2 and 5 extend parallel to each other and to the base 2a. In this way a simple yet stable holder 1 is obtained.

From a viewpoint of achieving an optimum stability of the bottle 10 in the holder 1, as shown in Figure 2, it is in particular preferable for the surface area of the second partial surface 5 to be at least as large as the surface area of the first partial surface 2, with the partial surface 5 preferably extending at the front side of the holder 1, seen in relation to the first partial surface 2.

Preferably, the holder 1 furthermore comprises a third partial surface 6, which functions to increase the stability of the holder. The third partial surface 6 and the second partial surface 5 are located on different sides of the first partial surface 2. More in particular, the surface area of the third partial surface 6 is smaller than the surface area of the second partial surface 5.

Another advantage of the holder according to the invention is the fact that the manufacturer of the lens cleaning fluid will have a possibility of providing one of the two upright walls 3 and 4, or both, with a commercial text (on the side of the bottle that is exposed to view). This makes it possible to produce the holder without any additional costs for the consumer.

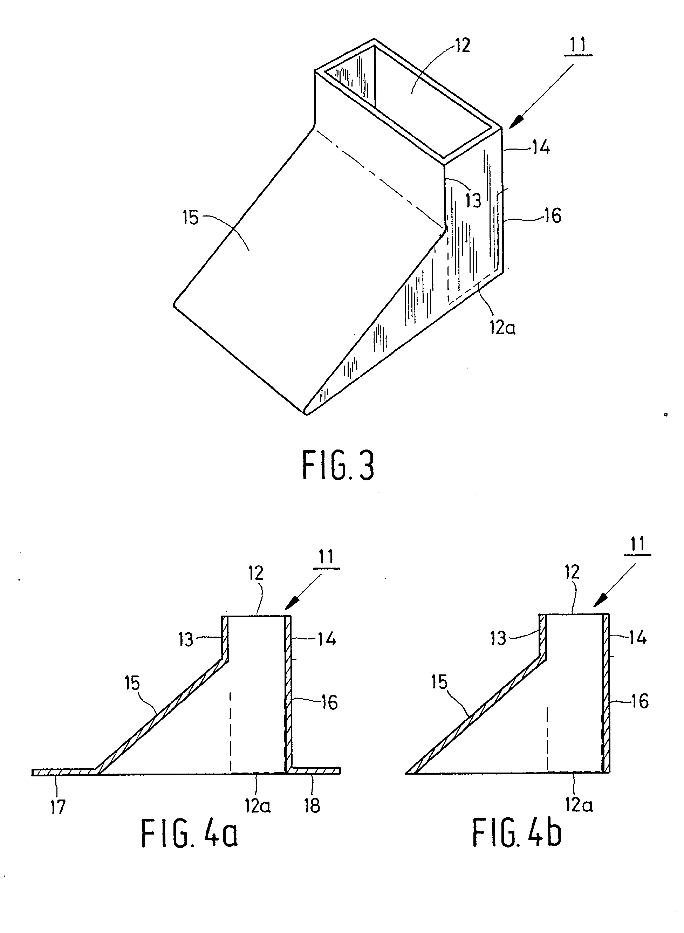

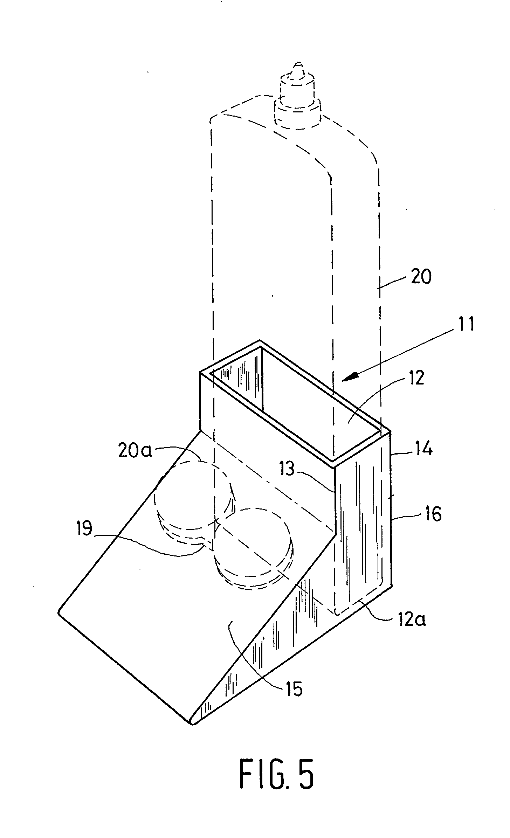

A second preferred embodiment is shown in Figure 3. In this embodiment, the first partial surface 12 is an opening into which the bottle 20 can be placed, which first partial surface 12 is spaced in vertical direction from the base 12a formed by the bottom of the bottle that is to be placed. This holder 11 gives the bottle 10 even greater stability, as is shown in Figure 5, since a larger part of the bottle 10 is enveloped by the holder 11.

The second partial surface 15 preferably extends at an angle with respect to the base 12a in order to bridge the difference in height between the base 12a and the first partial surface 12. The magnitude of said angle is not specifically limited, it may be a substantially straight angle or an oblique angle, as is shown in Figures 4a and 4b. The second partial surface may be straight or curved in any way that may be desired.

The present second embodiment may comprise an additional third partial surface 16 with a view to providing proper stability, which third partial surface 16 extends on another side of the first base portion 12 than the second partial surface 15.

The third partial surface 16 preferably extends at an angle to the base 12a in order to bridge the difference in height between the base 12a and the first partial surface 12. The magnitude of said angle is not specifically limited, it may be a substantially straight angle, for example, as is shown in Figures 4a and 4b.

In an especially preferred embodiment, an additional fourth partial surface 17 is provided, as is shown in Figure 4a, which partial surface extends on another side of the second partial surface 15 than the first partial surface 12. Said additional fourth partial surface 17 may provide additional stability for the bottle 20 when present in the holder 11, as is shown in Figure 5. Figure 4b shows the simplest variant of this embodiment, which does not comprise a fourth partial surface 17.

Furthermore preferably, said fourth partial surface 17 extends parallel to the base 12a, since this arrangement ensures the greatest degree of stability, because it provides additional support of the holder 11 as a whole.

In order to further increase the stability, an additional fifth partial surface 18 may be present, which partial surface extends on another side of the third partial surface 16 than the first partial surface 12, as is shown in Figure 4a. The simplest variant of the second embodiment according to the invention as shown in Figure 4b does not comprise such a fifth partial surface 18.

Preferably, said fifth partial surface 18 extends parallel to the base 12a, since this arrangement ensures the greatest degree of stability, because it provides additional support of the holder 11 as a whole.

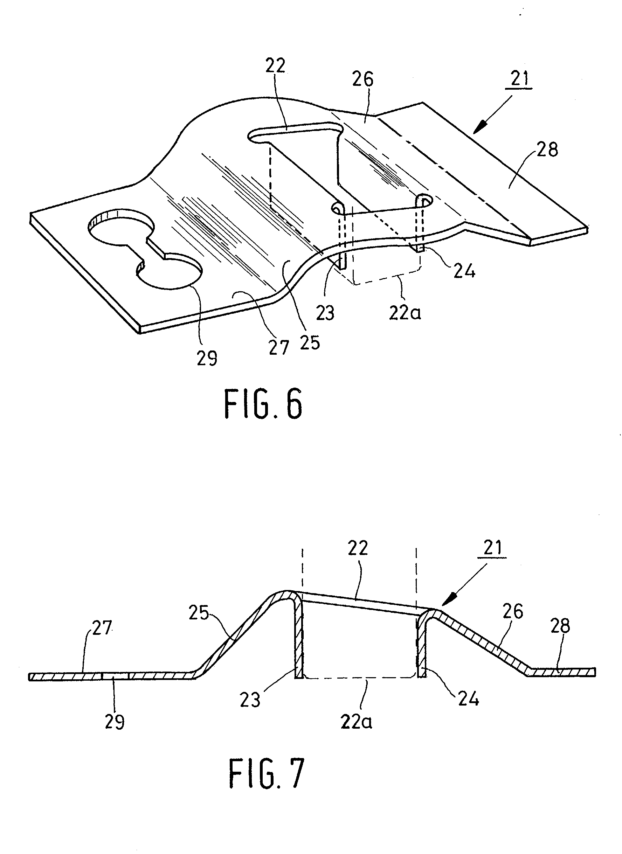

A third embodiment of the present invention is shown in Figures 6-8. In this embodiment, the second partial surface 25 and the third partial surface 26 may be arranged at an oblique angle with respect to the base 22a formed by the bottom of the bottle to be placed, as is shown in Figures 6 and 7.

In this third embodiment as shown in Figure 5, either one of the second and third partial surfaces 25 and 26, or both, may be straight or curved in any way that may be desired. Figure 7 shows a preferred embodiment, in which the second partial surface 25 and the third partial surface 26 are curved.

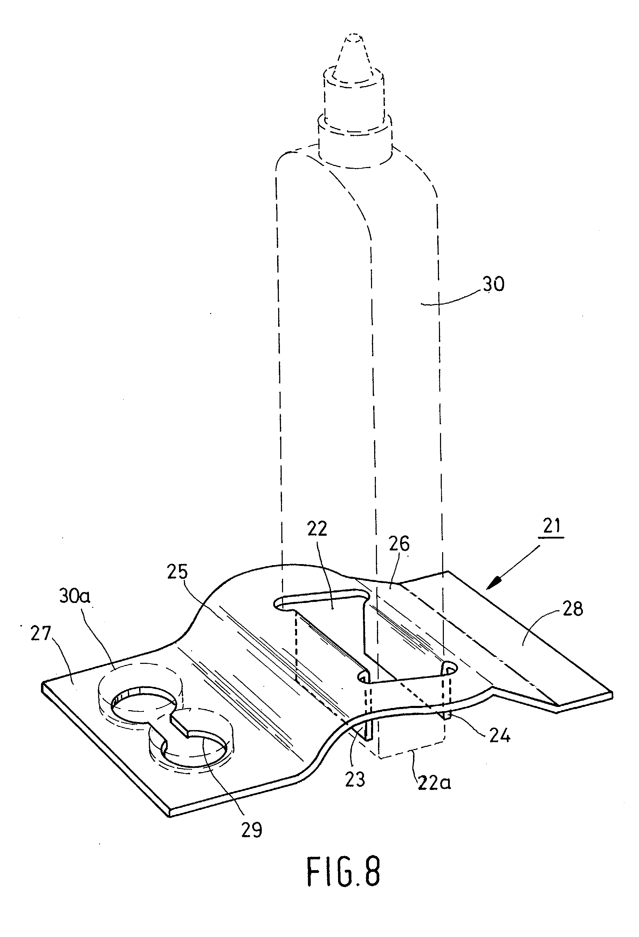

In an especially preferred embodiment, an additional fourth partial surface 27 is present, as is shown in Figure 7, which base portion extends on another side of the second partial surface 25 than the first partial surface 22. Said additional fourth partial surface 27 can provide additional stability for the bottle 30 when present in the holder 21 (Figure 8).

Preferably, said fourth partial surface 27 extends parallel to the base 22a, since this arrangement ensures the greatest degree of stability, because it provides additional support of the holder 21 as a whole.

In order to further increase the stability, an additional fifth partial surface 28 may be present, which partial surface extends on another side of the third partial surface 26 than the first partial surface 22, as is shown in Figure 7.

Preferably, said fifth partial surface 28 extends parallel to the base 22a, since this arrangement ensures the greatest degree of stability, because it provides additional support of the holder 11 as a whole.

As is shown in Figures 6 and 7, the walls 23, 24 preferably extend in downward direction in order to form a cavity in which the bottle can be secured.

Preferably, said walls extend substantially to the base 22a in order to bridge the difference in height between the first base portion 22 and the base 22a. In this way the largest possible contact area between the walls 23 and 24 on the one hand and the bottle 30 on the other hand is obtained, as is shown in Figure 8, thus providing the greatest degree of stability for the bottle 30.

Preferably, a contact lens case 10a,20a,30a is present on one or more of the partial surfaces 5,6,15,16,17,18,25,26,27,28 in all three of the embodiments as described above, as is shown in the respective Figures 2, 5 and 8. In this way, the contact lenses and the associated bottle 10,20,30 with fluid present therein can easily be stored together, so that the fluid will be directly available for cleaning the contact lenses.

To that end, one or more of the aforesaid partial surfaces may be provided with means in the form of slots or grooves or a notch (9,19,29). It is also possible to provide one or more of the aforesaid partial surfaces with a strip of Velcro, on which the contact lens case 10a,20a,30a can be placed. This is not shown in the Figures, however.

The advantage of placing the commercial text on the holder according to the second and the third embodiment of the invention is retained, so that the manufacturer of the contact lens fluid will have a possibility of placing a commercial text on one or more of the partial surfaces 5,6,15,16,17,18,25,26,27,28. This makes it possible to produce the holder without any additional costs for the consumer, since the costs involved in the manufacture of the holder can be paid from the advertising proceeds.

In addition to that, the height of at least one, or both, of the rigid upright walls 3+4, 13+14 and 23+24 may be such in all the embodiments that the bottle is secured against falling over.

A bottle can be stably placed between the rigid upright walls 3+4, 13+14 and 23+24 in the present holder, so as to store the bottle in an upright position and protect it against falling over. Since the walls are rigid, the stability is increased. The first partial surface 2,12,22 extends parallel to the base 2a,12a,22a, in particular in order to ensure that the bottle 10,20,30 as shown in Figure 2 is anchored in a substantially upright position in the holder 1,11,21. The surface area of the second partial surface 5,15,25 is such that it amounts to at least 50% of the surface area of the first partial surface 2,12,22. This minimum size contributes towards obtaining an excellent stability and prevents the bottle from falling over.

It will be understood that the simplest embodiments of the holder according to the invention have been designed for a specific bottle, viz. a bottle whose bottom has a specific shape. It is also possible, of course, for at least one of the upright walls (3+4, 13+14 and 23+24) to be movable with respect to the base, or for at least one upright wall to be movable over one or more of the above-mentioned partial surfaces. When using such a construction, which is more expensive, bottles having bottoms of slightly varying size can be clampingly placed on the holder according to the invention.

In addition to that it is preferable to provide at least part of one or more or the partial surfaces (15,16,17,18,25,26,27,28) with a reflective surface. Said reflective surface is very useful when placing the contact lens on the eye, so that no additional mirror will be needed.

It will be apparent to those skilled in this field that there are several obvious possibilities of modifying the holder for the bottle containing the fluid for cleaning contact lenses and the combination of a bottle and a holder, which modifications will fall within the scope of the invention.

## Claims

1. A holder for a bottle intended for containing a fluid to be used for cleaning contact lenses, which holder (1,11,21) defines at least two partial surfaces (2+5, 12+15, 22+25) and which comprises two upright walls (3+4, 13+14, 23+24), **characterized in that** the first partial surface (2,12,22) extends parallel to a base defined by the bottom of the bottle to be placed, wherein at least two rigid upright walls (3+4, 13+14, 23+24) extend from said first partial surface (2,12,22) for stabilising the bottle, and wherein the surface area of the second partial surface (5,15,25) amounts to at least 50% of the surface area of the first partial surface (2,12,22).

2. A holder according to claim 1, **characterized in that** the two partial surfaces (2,5) extend parallel to each other and to the base, wherein the first partial surface (2) is surrounded by the two rigid upright walls, which extend in upward direction from the base, for the purpose of increasing the stability of the bottle.

3. A holder according to any one or more of the preceding claims, **characterized in that** the surface area of the second partial surface (5) is at least as large as the surface area of the first partial surface (2).

4. A holder according to any one or more of the preceding claims, **characterized in that** the second partial surface (5) extends at the front side of the holder, seen in relation to the first partial surface (2).

5. A holder according to any one or more of the preceding claims, **characterized in that** a third partial surface (6) is provided, which third partial surface (6) extends on another side of the first partial surface (2) than the second partial surface (5).

6. A holder according to any one or more of the preceding claims, **characterized in that** one of the two rigid upright walls (3,4), or both, are provided with a commercial text.

7. A holder according to claim 1, **characterized in that** the first partial surface (12,22) is an opening for the bottle to be placed, which first partial surface (12,22) is spaced in vertical direction from the base.

8. A holder according to claim 7, **characterized in that** the second partial surface (15,25) extends at an angle with respect to the base in order to bridge the difference in height between the base and the first partial surface (12,22).

9. A holder according to any one of the claims 7-8, **characterized in that** a third partial surface (16,26) is provided, which third partial surface (16,26) extends on another side of the first partial surface (12,22) than the second partial surface (15,25).

10. A holder according to claim 9, **characterized in that** the third partial surface (16,26) extends at an angle with respect to the base in order to bridge the difference in height between the base and the first partial surface (12,22).

11. A holder according to any one or more of the claims 7-10, **characterized in that** a fourth partial surface (17,27) is provided, which fourth partial surface (17,27) extends on another side of the second partial surface (15,25) than the first partial surface (12,22).

12. A holder according to claim 11, **characterized in that** the fourth partial surface (17) extends parallel to the base.

13. A holder according to any one or more of the claims 9-12, **characterized in that** a fifth partial surface (18,28) is provided, which fifth partial surface (18,28) extends on another side of the third partial surface (16,26) than the first partial surface (12,22).

14. A holder according to claim 13, **characterized in that** the fifth partial surface (18) extends parallel to the base.

15. A holder according to any one or more of the claims 7 - 14, **characterized in that** at least two upright rigid walls (23+24) extend in downward direction from the first partial surface (22).

16. A holder according to claim 15, **characterized in that** said downwardly extending, upright rigid walls (23+24) extend substantially to the base in order to bridge the difference in height between the first partial surface (22) and the base.

17. A holder according to any one or more of the claims 1-16, **characterized in that** a recess (9,19,29) is present in one or more of the partial surfaces (5,6,15,16,17,18,25,26,27,28).

18. A holder according to any one or more of the claims 1-17, **characterized in that** a contact lens case (10a,20a,30a) is present on one or more of the partial surfaces (5,6,15,16,17,18,25,26,27,28).

19. A holder according to any one or more of the claims 7-18, **characterized in that** a commercial text is present on one or more of said partial surfaces (15,16,17,18,25,26,27,28).

20. A holder according to any one or more of the preceding claims, **characterized in that** the height of at least one of the rigid upright walls (3+4, 13+14 and 23+24) suffices to secure the bottle (10,20,30) against falling over.

21. A holder according to any one or more of the preceding claims, **characterized in that** at least one of the upright walls (3+4, 13+14 and 23+24) is movably disposed.

22. A holder according to any one or more of the preceding claims, **characterized in that** at least part of one or more or the partial surfaces (15,16,17,18,25,26,27,28) is provided with a reflective surface.

23. A combination of a holder and a bottle, which bottle (10,20,30) is intended for containing a fluid to be used for cleaning contact lenses, **characterized in that** said holder corresponds to a holder as defined in any one or more of the preceding claims.
